# EUROPÄISCHE PATENTSCHRIFT

(11) **EP 0 483 195 B1**
(45) Veröffentlichungstag und Bekanntmachung des Hinweises auf die Patenterteilung: **30.11.1994**
(21) Anmeldenummer: 90910703.9
(22) Anmeldetag: 09.07.1990
(51) Int. Cl.: C07C 219/06, C07C 213/06

(54) **VERFAHREN ZUR HERSTELLUNG VON QUARTÄREN AMMONIUMVERBINDUNGEN**
PROCESS FOR PREPARING QUATERNARY AMMONIUM COMPOUNDS
PROCEDE DE FABRICATION DE COMPOSES QUATERNAIRES DE L'AMMONIUM

(30) Priorität: 17.07.1989 ES 8902519
(43) Veröffentlichungstag der Anmeldung: 06.05.1992
(73) Patentinhaber: Henkel Kommanditgesellschaft auf Aktien, 40191 Düsseldorf (DE)
(72) Erfinder: TRIUS, Antonio, E-Valldoreix (Sant Cugat) (ES); HUMBERT, Miquel, E-08902 Sabadell (ES); BIGORRA, Joaquim, E-08203 Sabadell (ES); CUADRADO, Francisco, E-08013 Barcelona (ES); POMARES, Jesús, E-08028 Barcelona (ES)
(86) Internationale Anmeldenummer: EP9001111
(87) Internationale Veröffentlichungsnummer: WO9101295

(56) Entgegenhaltungen:
- EP-A- 0 069 948
- EP-A- 0 075 168
- EP-A- 0 295 385
- DE-A- 3 402 146
- FR-A- 2 054 337
- US-A- 3 915 867
- US-A- 4 789 491

## Beschreibung

Die vorliegende Erfindung betrifft ein Verfahren zur Herstellung von quartären Ammoniumverbindungen, die langkettige Fettsäure-Ester-Gruppen enthalten.

Die erfindungsgemäß hergestellten Verbindungen bieten mindestens drei Vorteile gegenüber den seit langen bekannten Verbindungen vom Typ Dimethyldistearylammoniumchlorid:
1. Schnellere biologische Abbaubarkeit
2. Besser Wiederbenetzbarkeit der damit behandelten Textilien
3. Die Möglichkeit zur Herstellung hochkonzentrierter Textilweichmacher-Rezepturen mit niedriger Viskosität.

Hinzu kommt, daß die erfindungsgemäß hergestellten Verbindungen weder toxisch noch schädlich sind und daß sie eine sehr geringe Primärreizung von Haut und Augen verursachen, wobei die biologische Abbaubarkeit durch die Esterfunktion gewährleistet ist (CESIO-Kongreß 1988, Bd. III, Seiten 415 bis 429).

Auch die Vermarktung und der Einsatz von konzentrierten Textilweichmacher-Rezepturen bieten gegenüber verdünnten Rezepturen verschiedene Vorteile:
a) Geringeres zu transportierendes Gewicht und Volumen
b) Verminderung der Abfüllkosten
c) Vergrößerung der Herstellkapazität
Mit den klassischen quartären Ammoniumverbindungen vom Typ Dimethyldistearylammoniumsalz ist es nicht möglich, stabile Dispersionen mit hoher Konzentration (zwischen 15 und 30 Gew.-% kationischer Aktivsubstanz) und niedriger Viskosität herzustellen. Mit den erfindungsgemäß hergestellten quartären Ammoniumverbindungen lassen sich dagegen stabile wäßrige Dispersionen niedriger Viskosität in einem sehr einfachen industriellen Rührverfahren erzeugen, wobei es nicht notwendig ist, die Homogenisierung unter Druck vorzunehmen und Erdalkalimetallsalze einzusetzen, die, obwohl sie zunächst die Viskosität der Dispersion herabsetzen, im Laufe der Zeit eine Erhöhung der Viskosität und Phasentrennung herbeiführen können.

Weitere gewünschte Eigenschaften von Textilweichmacher-Rezepturen sind eine helle Farbe des Wirkstoffs und eine Vergilbungsbeständigkeit des mit diesem Wirkstoff behandelten Gewebes. Diese Eigenschaften weisen die Textilweichmacher-Rezepturen mit erfindungsgemäß hergestellten quartären Ammoniumverbindungen auf.

Die erfindungsgemäß hergestellten Produkte sind an sich bekannt. In der US-Patentschrift 3,915,867 aus dem Jahre 1975 werden diese quartären Ammoniumverbindungen beschrieben. Man erhält diese Produkte durch Umesterung einer bestimmten Mischung von Palmfettsäure- und Talgfettsäuremethylester mit Triethanolamin und nachfolgender Quarternisierung mit Dimethylsulfat. Die so hergestellten quartären Ammoniumverbindungen weisen die wünschenswerten Eigenschaften auf. Für ihre Herstellung ist es jedoch notwendig, einen verhältnismäßig edlen Rohstoff zu verwenden, wie ihn die speziellen Palmfettsäure- und Talgfettsäuremethylester erhöhter Reinheit darstellen; dieser Rohstoff ist darüberhinaus wenig lagerstabil, was dazu führt, daß manchmal quartäre Ammoniumverbindungen mit dunkler Farbe erhalten werden.

Es sind auch Vorschläge gemacht worden zur Herstellung der quartären Ammoniumverbindungen, Fettsäure direkt mit unterschiedlichen Hydroxyalkylaminen zu verestern (Deutsche Patentanmeldungen Nr. 2 727 841, 3 127 239, 3 137 043, 3 402 146) und Süd-Afrikanische Patentanmeldung Nr. 6701747. Mit keinem der dort beschriebenen Verfahren erhält man allerdings einen Textilweichmacher, der hinsichtlich Reinheit, Farbe und Geruch sowie Lagerstabilität den Anforderungen entspricht.

Aus EP-A-75 168 ist ein Verfahren zur Herstellung von Ammoniumverbindungen durch Alkylierung von Aminen mit Estern der phosphorigen Säure und der Phosphorsäure bekannt.

Die vorliegende Erfindung geht daher von der Aufgabe aus, quartäre Ammoniumverbindungen mit Esterfunktion herzustellen, für deren Herstellungsverfahren ein leicht zugänglicher und preiswerter Rohstoff verwendet werden kann. Hierfür bot sich die Direktveresterung einer Fettsäure mit einem Polyhydroxyalkylamin, wie beispielsweise das Triethanolamin an. Ein Komplex von Reduktionsmitteln stellte sich als ein geeigneter Katalysator heraus. Dadurch erreichte man nicht nur, daß die Veresterungszeit abgekürzt werden konnte, sondern man erhielt sogar Endprodukte mit heller Farbe und geringem Eigengeruch, die zudem auch noch einen hohen Gehalt an Diestern (60 bis 80 Gew.-%) aufweisen. Dadurch läßt sich das anwendungstechnische Verhalten der mit diesen Produkten hergestellten Weichmacher-Rezeptur wesentlich verbessern.

Gegenstand der vorliegenden Erfindung ist daher ein Verfahren zur Herstellung von kationischen quartären Ammoniumverbindungen mit einer Esterfunktion der folgenden allgemeinen Formel:

R¹R²R³N⁺R⁴ A⁻;

wobei R¹, R², R³ entweder
- eine Alkylgruppe der Formel CₙH₂ₙ₊₁, wobei n jeweils 1 bis 4 ist, oder
- eine Hydroxyalkylgruppe oder eine Polyhydroxyalkylgruppe der Formel CₙH_{(2n+1-x)(OH)x}, wobei n und x jeweils 1 bis 4 sind, oder
- eine Estergruppe der Formel R⁵COOR⁶-, wobei R⁵ eine Alkyl- oder eine Alkenylgruppe mit 6 bis 22 Kohlenstoffatomen und R⁶ eine Gruppe der Formel 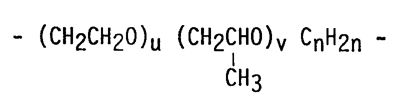 sind, wobei n jeweils 1 bis 4, u und v jeweils 0 bis 6 in beliebiger Anordnung sind; R⁴ ist
- eine Alkylgruppe der Formel CₙH₂ₙ₊₁, wobei n jeweils 1 bis 4 ist
- eine Polyoxyalkylenkette der Formel 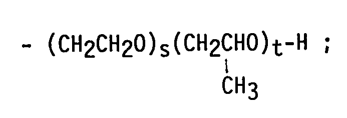 wobei s und t jeweils 0 bis 6 sind; A⁻ ist ein Gegenanion, das wahlweise
- Alkylsulfat der Formel SO₄(CₙH₂ₙ₊₁)⁻, wobei n jeweils 1 bis 4 ist;
- Alkylphosphat der Formel PO₄ (CₙH₂ₙ₊₁)₂⁻, wobei n jeweils 1 bis 4 ist;
- das Anion einer organischen Säure der Formel CₙH_{(2n-1-a-b)} (OH)ₐ (COOH)_{b}, wobei n jeweils 1 bis 4 und a, b jeweils 1 bis 3 sind;
- ein Phosphat- oder Phosphit-Anion; Cl⁻, Br⁻, CH₃C₆H₄SO₃⁻;
   wobei das Verfahren dadurch gekennzeichnet ist, daß es die folgenden Schritte enthält:
   a) Veresterung einer Fettsäure der Formel R₅COOH mit einem Alkanolamin der Formel R¹R²NR⁶-OH in Anwesenheit eines Reduktionsmittels aus der Gruppe unterphosphorige Säure, phosphorige Säure, Oxalsäure oder deren Alkali- oder Erdalkalimetall-Salze oder deren organische Ester oder eines organischen Reduktionsmittels aus der Gruppe Thioacetamin, Hydrazin, Hydrochinon und deren Derivate, unter anschließendem Durchleiten eines Luftstroms durch den Ester mit einer Temperatur von 40 bis 50 °C und einem Verhältnis von Luft zu Ester im Bereich von 10 : 1 bis 0,5 : 1, vorzugsweise in einem Verhältnis von 3 : 1 m³ Luft pro t Ester durch das Reaktionsgemisch; und
   b) Alkylierung der erhaltenen Verbindung durch ein Reagens der Formel R⁴ A oder durch ein Alkylenoxid aus der Gruppe Ethylenoxid, Propylenoxid oder deren Mischungen.

Zu den beiden Verfahrensschritten wird folgendes ausgeführt:

### Schritt A: Veresterung

Es erfolgt die Veresterung einer aliphatischen Fettsäure mit 6 bis 22 Kohlenstoffatomen in ihrer Kohlenwasserstoffkette, vorzugsweise mit 12 bis 22 Kohlenstoffatomen, mit einem Hydroxyalkylamin, das mindestens ein dreiwertiges aminartiges Stickstoffatom besitzt. Dieses Amin kann vorzugseise ein Methyldiethanolamin oder ein Triethanolamin oder eines seiner Derivate sein sowie deren Kondensationsprodukte mit einem oder mehreren Ethylenoxid- und/oder Propylenoxidmolekülen.

Die Veresterung führt man im Temperaturbereich von 120 bis 240°C, vorzugsweise zwischen 140 und 200°C, und insbesondere zwischen 160 und 180°C durch. Um das durch die Veresterung frei werdende Wasser zu beseitigen, kann ein Vakuum zwischen 1 und 100 Torr und vorzugsweise zwischen 1 und 20 Torr, und insbesondere zwischen 1 und 5 Torr angelegt werden. Eine andere bevorzugte Ausführungsform der vorliegenden Erfindung geht davon aus, daß man die Veresterung bei Atmosphärendruck in Anwesenheit eines inerten Lösungsmittels durchführt. Als inertes Lösungsmittel verwendet man vorzugsweise einen aliphatischen oder einen aromatischen Kohlenwasserstoff, wie z. B. Toluol, Xylol, n-Heptan, n-Decan. Die Veresterung führt man insbesondere in Anwesenheit eines sauren Katalysators, z. B. einer Sulfonsäure wie p-Toluolsulfonsäure oder Methansulfonsäure oder Phosphorsäure, phosphorige Säure oder unterphosphorige Säure oder Oxalsäure oder einer Lewis-Säure, wie z. B. Zinn-II-Salzen durch. Auch Alkalimetall- oder Erdalkalimetallsalze der zuletzt genannten Säuren oder deren organische Ester oder organische Reduktionsmittel aus der Gruppe Thioacetamin, Hydrazin, Hydrochinon und deren Derivate sind bevorzugte Reduktionsmittel für die Durchführung der Veresterung. Vorzugsweise wird als Katalysator unterphosphorige Säure eingesetzt, die gleichzeitig als Reduktionsmittel fungiert, oder aber Kombinationen aus dieser Säure oder deren Salze mit unterschiedlichen anderen sauren Katalysatoren; dadurch erreicht man Endprodukte mit besserer Farbe, und die Veresterung führt zu Derivaten mit einem hohen Anteil an Diestern.

Bei der Veresterung unter Verwendung von Katalysatoren wie phosphoriger Säure, unterphosphoriger Säure, deren Salze oder möglichen Kombinationen mit anderen sauren Katalysatoren ist es ratsam, einen Luftstrom durch das Reaktionsgemisch zu leiten, wodurch das Auftreten von Zersetzungsprodukten der Phosphorderivate mit unangenehmen Geruch im Endprodukt vermieden werden kann. Der durchzuleitende Luftstrom hat eine Temperatur von 40 bis 50°C; man leitet ihn in einem Verhältnis Luft zu Ester im Bereich von 10 : 1 bis 0,5 : 1, vorzugsweise in einem Verhältnis von 3 : 1 m³ Luft pro t Ester durch das Reaktionsgemisch. Man erhält hierdurch vollständig geruchlose Endprodukte mit guter Farbe.

Die einzusetzenden Fettsäuren können natürlichen oder synthetischen Ursprungs sein. Die Fettsäuren natürlichen Ursprungs leiten sich von Olivenöl, Sojaöl, Sonnenblumenöl, Palmöl, Rapsöl, Rizinusöl, Sesamöl, Talg, Fischöl, usw. ab, wobei auch deren hydrierte Derivate Verwendung finden können. Weitere Fettsäuren, die für das erfindungsgemäße Verfahren geeignet sind, sind Caprinsäure, Caprylsäure, Capronsäure, Laurinsäure, Valeriansäure, Myristinsäure, Palmitinsäure, Stearinsäure, Ölsäure, Elaidinsäure, Linolsäure, Linolensäure, Behensäure, Erucasäure und Rizinusölsäure. Bei den Fettsäuren synthetischen Ursprungs sind alle vorgenannten geradkettigen Komponenten sowie die verzweigtkettigen Typen erwähnenswert, wie beispielsweise solche, die aus der Oxo-Synthese stammen und Isostearinsäure oder aber Derivate aus der Guerbet-Kondensation.

Die Verwendung von hydrierten Fettsäuren führt zu einer besseren Weichmachungsleistung als die Verwendung der entsprechenden ungesättigten Fettsäuren, wobei die zuletzt genannten jedoch ein besseres Wiederbenetzungsvermögen aufweisen. Durch den gezielten Einsatz von gesättigten und ungesättigten Fettsäuren läßt sich ein ausgewogenes Verhältnis zwischen Weichmachungsleistung und Wiederbenetzungsvermögen einstellen.

Entsprechendes gilt für die Kettenlänge der Fettsäuren, wo zunehmende Kettenlänge zu einer verbesserten Weichmachungsleistung und abnehmende Kettenlänge zu einem verbesserten Wiederbenetzungsvermögen führt.

Das einzusetzende Amin kann ein Alkanolamin vom Typ Monomethyl-, Monoethyl-, Monopropyl- oder Monoisopropylethanolamin sein, an das gegebenenfalls Ethylenoxid, Propylenoxid oder eine Kombination von beiden im Gesamtverhältnis Alkylenoxid/Amin von 1 : 5, vorzugsweise 2 : 4, kondensiert ist. Das einzusetzende Amin kann auch vom Typ Dimethyl-, Diethyl-, Dipropyl- oder Diisopropylethanolamin, kondensiert mit Ethylenoxid, Propylenoxid oder einer Kombination von beiden im Gesamtverhältnis Alkylenoxid/Amin von 1 : 5, vorzugsweise 1 : 3 sein. Weitere geeignete Amine sind Triethanolamin, Tripropanolamin oder Triisopropanolamin oder deren Kondensationsprodukte mit Ethylenoxid, Propylenoxid oder einer Kombination von beiden im Gesamtverhältnis Ethylenoxid/Amin von 1 : 5, vorzugsweise 1 : 2.

Bezogen auf das Reaktionsgemisch setzt man an Reduktionsmittel und Katalysator 0,01 bis 5 Gew.-%, vorzugsweise 0,02 bis 0,5 Gew.-%, und insbesondere 0,05 bis 0,15 Gew.-% ein.

Bevorzugte Amine sind Triethanolamin, Methyldiethanolamin oder deren Anlagerungsprodukte mit Ethylenoxid und/oder mit Propylenoxid. Je nach Wahl der Reaktionsbedingungen erhält man Produkte mit einem unterschiedlich hohem Gehalt an Diester. Produkte mit einem Gehalt an Diester im Bereich von 60 bis 80 Gew.-% haben besonders wertvolle Eigenschaften. Es ist daher bevorzugt, die Reaktionsbedingungen so einzustellen, daß das Reaktionsgemisch einen Gehalt an Diestern von 60 bis 80 % hat. Bei dem erfindungsgemäßen Verfahren stellt man das Verhältnis von Fettsäure zu Amin so ein, daß es im Bereich von 0,2 : 1 bis 1 : 1, vorzugsweise von 0,4 : 1 bis 0,8 : 1 und insbesondere im Bereich von 0,5 : 1 bis 0,7 : 1 ein.

Als Alkylierungsreagens verwendet man zweckmäßigerweise ein Dialkylsulfat, vorzugsweise Dimethylsulfat.

### Schritt B: Alkylierung

Der durch die Veresterungsreaktion erhaltene Ester bzw. das Estergemisch wird bei einer Temperatur zwischen 30 und 100°C, vorzugsweise zwischen 40 und 80°C und insbesondere zwischen 40 und 60°C alkyliert, und zwar entweder in Anwesenheit oder in Abwesenheit eines kurzkettigen aliphatischen Alkohols, vorzugsweise Ethylalkohol oder Isopropylalkohol. Möglich ist auch die Alkylierung in Gegenwart eines Gemischs aus den genannten Alkoholen mit Wasser. Die Konzentration der oben genannten Verdünnungsmittel liegt dabei zweckmäßigerweise im Bereich von 5 bis 50 Gew.-%, vorzugsweise von 10 bis 30 Gew.-% und insbesondere im Bereich von 15 bis 25 Gew.-%, bezogen auf das Reaktionsgemisch. Im Fall der Verwendung einer Mischung aus Alkohol und Wasser als Verdünner liegt das Gewichtsverhältnis im Bereich von 1 : 4 bis 4 : 1.

Je nach dem eingesetzten Alkylierungsmittel wird die Alkylierung bei Normaldruck oder bei Drücken zwischen 2 und 11 bar, vorzugsweise zwischen 2 und 7 bar und insbesondere zwischen 2 und 5 bar durchgeführt.

Das Alkylierungsmittel setzt man in einer Menge von 0,5 bis 1, vorzugsweise von 0,8 bis 1 und insbesondere in einer Menge von 0,9 bis 0,95 Mol je Äquivalent an dreiwertigem aminartigem Stickstoff im Estergemisch ein. Als Alkylierungsmittel kann auch ein Alkylenoxid aus der Gruppe Ethylenoxid, Propylenoxid oder deren Mischungen verwendet werden. In diesem Fall ist es zweckmäßig, daß man das Alkylierungsmittel und den dreiwertigen aminartigen Stickstoff im Estergemisch im Verhältnis von 1 : 1 bis 20 : 1, vorzugsweise von 1 : 1 bis 6 : 1 und insbesondere im Bereich von 2 : 1 bis 4 : 1 ein. Dabei ist es zweckmäßig, daß man die Alkylierungsreaktion bei einem Druck zwischen 1 und 10 bar, vorzugsweise zwischen 1 und 6 bar und insbesondere zwischen 2 und 4 bar durchführt.

### Beispiele

### Beispiel 1:

### Veresterung

In einem beheizbaren Labordestilierkolben mit Destillations- und Kühlvorrichtung sowie Vakuumanschluß, der 1 Torr erreicht, wurden 540 g Talgfettsäure (2 Mol), 149 g Triethanolamin (1 Mol) und 1,4 g 50%ige unterphosphorige Säure gegeben. Über einen Zeitraum von 1 bis 2 Stunden wurde die Temperatur des Reaktors auf 160°C gebracht, während das Vakuum gleichzeitig nach und nach auf 30 Torr eingestellt wurde. Diese Bedingungen wurden solange beibehalten, bis die Säurezahl der Masse unter 5 mg KOH pro g lag (Bestimmung der Lösung in einer Ethylalkoholprobe mit Kaliumhydroxid, 0,1 N und Phenolphthalein als Indikator). Sobald dieser Wert erreicht war (3 bis 4 Stunden) wurde die Masse unter Aufrechterhaltung des Vakuums auf 40 bis 50°C abgekühlt und das Vakuum anschließend mit Stickstoff verringert, bis der atmosphärische Druck erreicht war.

Anschließend wurde unter ständigem Rühren ein Liter Luft innerhalb von 15 Minuten durch den Ester geleitet. Der Gehalt an Diester (ermittelt durch Hochdruckflüssigkeits-Chromatographie), betrug 62 %. Das Verhältnis von Mono-, Di- und Triestern betrug 10 % : 62 % : 28 %.

### Alkylierung

Dem obigen Estergemisch wurden 137 g Isopropylalkohol zugesetzt, und unter Aufrechterhaltung der Temperatur von 40 bis 50°C wurden 120 g Dimethylsufat über einen Zeitraum von 2 Stunden hinzugegeben. Die Masse wurde weitere 2 Stunden bei 60°C gerührt. Man erhielt ein Produkt mit einem Gehalt an Trockensubstanz (Rückstand nach 2 Stunden bei 105°C) von 85 Gew.-% und einer Farbe nach Gardner von unter 1.

### Beispiel 2:

### Veresterung

In einer Laboreinrichtung ähnlich der in Beispiel 1 genannten, wurden 540 g Talgfettsäure (2 Mol), 149 g Triethanolamin (1 Mol), 0,7 g Methansulfonsäure und 0,7 g Natriumhypophosphit (PO₂H₂ Na . 2H₂O) gegeben. Über einen Zeitraum von 1 bis 2 Stunden wurde die Temperatur des Reaktors auf 140°C gebracht, während das Vakuum gleichzeitig nach und nach auf 30 Torr eingestellt wurde.

Diese Bedingungen wurden solange beibehalten, bis die Säurezahl des Reaktionsgemischs unter 5 mg KOH pro g lag. Sobald dieser Wert erreicht war (2 bis 3 Stunden), wurde die Masse unter Aufrechterhaltung des Vakuums auf 40 bis 50°C abgekühlt und das Vakuum anschließend mit Stickstoff abgebaut, bis atmosphärischer Druck erreicht war. Anschließend wurde innerhalb von 15 Minuten 1 Liter Luft unter ständigem Rühren durch den Ester geleitet. Zum Schluß wurde alkyliert, wie in Beispiel 1 beschrieben.

### Beispiel 3 (zum Vergleich):

### Veresterung

Es wurde unter Veresterungsbedingungen wie in Beispiel 1 gearbeitet, ohne daß Luft durch den Ester geleitet wurde. Folgende Mengen wurden eingesetzt:
540 g Talgfettsäure, 149 g Triethanolamin, 0,7 g Zinn-II-laurat. Das Verhältnis von Mono-, Di- und Triester (ermittelt mit Hochdruckflüssigkeits-Chromatographie) betrug 24 Gew.-% : 50 Gew.-% : 26 Gew.-%.

### Alkylierung

Das Estergemisch wurde unter den gleichen Bedingungen und mit den gleichen Reagenzien wie in Beispiel 1 quarterniert; man erhielt ein Endprodukt mit einer Gardner-Farbzahl von 5.

### Beispiel 4:

Für die Veresterung wurden dieselben Reagenzien und für die Alkylierung dieselben Bedingungen und Reagentien wie in Beispiel 1 eingesetzt; das Vakuum bei der Veresterung betrug jedoch 2 Torr.

### Beispiel 5:

Für die Veresterung wurden dieselben Reagenzien und für die Alkylierung dieselben Bedingungen und Reagenzien wie in Beispiel 1 eingesetzt; bei der Veresterung arbeitete man jedoch mit atmosphärischem Druck unter Einsatz von 50 g n-Heptan, um das sich bildende Wasser azeotrop abzuscheiden.

### Beispiel 6:

Für die Veresterung wurden dieselben Bedingungen und für die Alkylierung dieselben Bedingungen und Reagenzien wie in Beispiel 1 eingesetzt; die Talgfettsäure wurde jedoch durch hydrierte Fischfettsäure ersetzt.

### Beispiel 7:

Für die Veresterung wurden dieselben Bedingungen und für die Alkylierung dieselben Bedingungen und Reagenzien wie in Beispiel 1 eingesetzt; die Talgfettsäure wurde jedoch durch hydrierte Talgfettsäure ersetzt.

### Beispiel 8:

Für die Veresterung wurden dieselben Bedingungen und für die Alkylierung dieselben Bedingungen und Reagenzien wie in Beispiel 1 eingesetzt; die Talgfettsäure wurde jedoch durch Ölsäure ersetzt.

### Beispiel 9:

Für die Veresterung wurden dieselben Bedingungen und für die Alkylierung dieselben Bedingungen und Reagenzien wie in Beispiel 1 eingesetzt; die Talgfettsäure wurde jedoch durch partiell hydrierte Talgfettsäure ersetzt.

### Beispiel 10:

Für die Veresterung wurden dieselben Bedingungen und für die Alkylierung dieselben Bedingungen und Reagenzien wie in Beispiel 1 eingesetzt; die 149 g Triethanolamin wurden jedoch durch 237 g kondensiertes Triethanolamin mit 2 Mol Ethylenoxid ersetzt.

### Beispiel 11:

Für die Veresterung wurden dieselben Bedingungen und für die Alkylierung dieselben Bedingungen und Reagenzien wie in Beispiel 1 eingesetzt; die 149 g Triethanolamin wurden jedoch durch 265 g kondensiertes Triethanolamin mit 2 Mol Propylenoxid ersetzt.

### Beispiel 12:

Für die Alkylierung wurden dieselben Bedingungen und für die Veresterung dieselben Bedingungen und Reagenzien wie in Beispiel 1 eingesetzt; die 120 g Dimethylsulfat wurden jedoch durch 146 g Diethylsulfat ersetzt.

### Beispiel 13:

Für die Veresterung wurden dieselben Bedingungen und Reagenzien wie in Beispiel 1 eingesetzt; die Alkylierung wurde jedoch wie folgt durchgeführt:

In einen für einen Druck von 11 bar geeigneten Laborreaktor wurde die dem Beispiel 1 entsprechende Menge Estergemisch gegeben; hinzugegeben wurden 140 g Isopropylalkohol, 140 g Wasser und 98 g Phosphorsäure. Anschließend wurden innerhalb von 2 Stunden 88 g Ethylenoxid bei einem Druck von 5 bar und einer Temperatur von 80°C hinzugegeben. Das Ergebnis war ein Produkt mit einem Gehalt an 75 Gew.-% Trockensubstanz und einer Gardner-Farbzahl von unter 1.

### Beispiel 14:

Es wurden dieselben Bedingungen und Reagenzien wie in Beispiel 1 für die Veresterung und dieselben Bedingungen für die Alkylierung wie in Beispiel 13 eingesetzt, die 88 g Ethylenoxid wurden jedoch durch 16 g Propylenoxid ersetzt und dessen Dosierzeit auf 4 Stunden erhöht.

### Beispiel 15 (zum Vergleich):

Für die Veresterung wurden dieselben Bedingungen und für die Alkylierung dieselben Bedingungen und Reagenzien wie in Beispiel 1 eingesetzt; es wurde aber keine Luft durch das Estergemisch geleitet, wodurch man ein kationisches Produkt mit einer Gardner-Farbzahl von unter 1 erhielt; das Endprodukt wies einen unangenehmen Geruch auf.

### Beispiel 16 (zum Vergleich):

Für die Veresterung wurden dieselben Bedingungen und für die Alkylierung dieselben Bedingungen und Reagenzien wie in Beispiel 1 eingesetzt; anstelle von Luft wurde aber ein Stickstoffstrom durch das Estergemisch geleitet, wodurch man ein kationisches Endprodukt mit einer Gardner-Farbzahl von unter 1 erhielt; allerdings wies das Produkt einen unangenehmen Geruch auf.

### Beispiel 17 (zum Vergleich):

### Veresterung

In eine Laboreinrichtung ähnlich der in Beispiel 1 genannten wurden 568 g Talgfettsäuremethylester (2 Mol), 149 g Triethanolamin (1 Mol), 14 g Natriummethylat - 30 %ig in Methanol - gegeben. In einem Zeitraum von 1 bis 2 Stunden wurde der Reaktor auf 110°C gebracht und das Vakuum auf 30 Torr eingestellt. Diese Bedingungen wurden solange aufrechterhalten, bis der Gehalt an Methylester unter 2,5 Gew.-% lag (Ermittlung durch Dünnschicht-Chromatographie). Nachdem dieser Wert erreicht war (4 bis 5 Stunden), wurde die Masse unter Aufrechterhaltung des Vakuums auf 40 bis 50°C abgekühlt und das Vakuum anschließend mit Stickstoff abgebaut, bis Atmosphärendruck erreicht war.

Das Verhältnis von Mono-, Di- und Triester (gemessen mit Hochdruckflüssigkeits-Chromatographie) betrug 17 Gew.-% : 40 Gew.-% : 43 Gew.-%.

### Alkylierung

Das Estergemisch wurde unter denselben Bedingungen und mit denselben Reagenzien wie in Beispiel 1 quartärniert, wodurch man ein kationisches Endprodukt mit einer Gardner-Farbzahl von 9 bis 10 erhielt.

## Patentansprüche

1. Verfahren zur Herstellung von wenigstens eine Estergruppe enthaltenden kationischen quartären Ammoniumverbindungen der folgenden allgemeinen Formel:
R¹R²R³N⁺R⁴ A⁻;
wobei R¹, R², R³ entweder
- eine Alkylgruppe der Formel CₙH₂ₙ₊₁, wobei n jeweils 1 bis 4 ist, oder
- eine Hydroxyalkylgruppe oder eine Polyhydroxyalkylgruppe der Formel CₙH_{(2n+1-x)(OH)x}, wobei n und x jeweils 1 bis 4 sind, oder
- eine Estergruppe der Formel R⁵COOR⁶ -, wobei R⁵ eine Alkyl- oder oder eine Alkenylgruppe mit 6 bis 22 Kohlenstoffatomen und R⁶ eine Gruppe der Formel 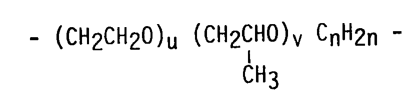 sind, wobei n jeweils 1 bis 4, u und v jeweils 0 bis 6 in beliebiger Anordnung sind; R⁴ ist
- eine Alkylgruppe der Formel CₙH₂ₙ₊₁, wobei n jeweils 1 bis 4 ist,
- eine Polyoxyalkylenkette der Formel 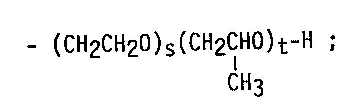 wobei s und t jeweils 0 bis 6 sind; A⁻ ist ein Gegenanion, das wahlweise
- Alkylsulfat der Formel SO₄(CₙH₂ₙ₊₁)⁻, wobei n jeweils 1 bis 4 ist;
- Alkylphosphat der Formel PO₄ (CₙH₂ₙ₊₁)₂⁻, wobei n jeweils 1 bis 4 ist;
- das Anion einer organischen Säure der Formel CₙH_{(2n-1-a-b)} (OH)ₐ (COOH)_{b}, wobei n jeweils 1 bis 4 und a, b jeweils 1 bis 3 sind;
- ein Phosphat- oder Phosphit-Anion;
- Cl⁻, Br⁻, CH₃C₆H₄SO₃⁻;
wobei das Verfahren dadurch gekennzeichnet ist, daß es die folgenden Schritte enthält:
a) Veresterung einer Fettsäure der Formel R₅COOH mit einem Alkanolamin der Formel R¹R²NR⁶-OH in Anwesenheit eines Reduktionsmittels aus der Gruppe unterphosphorige Säure, phosphorige Säure, Oxalsäure oder deren Alkali- oder Erdalkalimetall-Salze oder deren organische Ester oder eines organischen Reduktionsmittels aus der Gruppe Thioacetamin, Hydrazin, Hydrochinon und deren Derivate, unter anschließendem Durchleiten eines Luftstroms durch den Ester mit einer Temperatur von 40 bis 50 °C und einem Verhältnis von Luft zu Ester im Bereich von 10 : 1 bis 0,5 : 1, vorzugsweise in einem Verhältnis von 3 : 1 m³ Luft pro t Ester durch das Reaktionsgemisch; und
b) Alkylierung der erhaltenen Verbindung durch ein Reagens der Formel R⁴ A oder durch ein Alkylenoxid aus der Gruppe Ethylenoxid, Propylenoxid oder deren Mischungen.

2. Verfahren nach Anspruch 1, dadurch gekennzeichnet, daß man die Veresterung bei einer Temperatur zwischen 120 und 240°C, vorzugsweise zwischen 140 und 200°C, und insbesondere zwischen 160 und 180°C durchführt.

3. Verfahren nach Anspruch 1, dadurch gekennzeichnet, daß man die Veresterung bei einem Druck zwischen 1,333 und 133,3 mbar, vorzugsweise zwischen 1,333 und 26,66 mbar und insbesondere zwischen 1,333 und 6,665 mbar durchführt.

4. Verfahren nach Anspruch 1, dadurch gekennzeichnet, daß man die Veresterung bei Atmosphären-Druck in Anwesenheit eines inerten Lösungsmittels durchführt.

5. Verfahren nach Anspruch 4, dadurch gekennzeichnet, daß man als inertes Lösungsmittel einen aliphatischen oder einen aromatischen Kohlenwasserstoff, wie z. B. Toluol, Xylol, n-Heptan oder n-Decan verwendet.

6. Verfahren nach Anspruch 1, dadurch gekennzeichnet, daß man die Veresterung in Anwesenheit eines sauren Katalysators, z. B. einer Sulfonsäure wie p-Toluolsulfonsäure oder Methansulfonsäure oder Phosphorsäure, phosphorige Säure oder unterphosphorige Säure oder Oxalsäure oder eine Lewis-Säure wie z. B. Zinn-II-Salze vornimmt.

7. Verfahren nach Anspruch 6, dadurch gekennzeichnet, daß man, bezogen auf das Reaktionsgemisch, an Reduktionsmittel und Katalysator 0,01 bis 5 Gew.-%, vorzugsweise 0,02 bis 0,5 Gew.-%, und insbesondere 0,05 bis 0,15 Gew.-% verwendet.

8. Verfahren nach Anspruch 1, dadurch gekennzeichnet, daß man Fettsäuren natürlichen oder synthetischen Ursprungs oder aber deren Hydrierungs- oder Spaltprodukte verwendet.

9. Verfahren nach Anspruch 1, dadurch gekennzeichnet, daß man als Amin Triethanolamin, Methyldiethanolamin oder deren Anlagerungsprodukte mit Ethylenoxid und/oder mit Propylenoxid verwendet.

10. Verfahren nach Anspruch 1, dadurch gekennzeichnet, daß man die Reaktion so führt, daß das Reaktionsgemisch einen Gehalt an Diester von 60 bis 80 Gew.-% hat.

11. Verfahren nach Anspruch 1, dadurch gekennzeichnet, daß man als Alkylierungsreagens ein Dialkylsulfat oder ein Trialkylsulfat, vorzugsweise Dimethylsulfat verwendet.

12. Verfahren nach Anspruch 1, dadurch gekennzeichnet, daß man Fettsäure und Amin im Verhältnis von 0,2 : 1 bis 1 : 1, vorzugsweise von 0,4 : 1 bis 0,8 : 1 und insbesondere von 0,5 : 1 bis 0,7 : 1 je Äquivalent Alkanolamin verwendet.

13. Verfahren nach Anspruch 11, dadurch gekennzeichnet, daß man die Alkylierung bei Atmosphären-Druck in Anwesenheit eines kurzkettigen aliphatischen Alkohols, vorzugsweise Isopropylalkohol, der in einer Menge von 5 bis 50 Gew.-%, vorzugsweise von 10 bis 30 Gew.-%, und insbesondere von 15 bis 25 Gew.-%, bezogen auf das Reaktionsgemisch, durchführt.

14. Verfahren nach Anspruch 11, dadurch gekennzeichnet, daß man das Alkylierungsmittel in einer Menge von 0,5 bis 1, vorzugsweise von 0,8 bis 1 und insbesondere in einer Menge von 0,9 bis 0,95 mol je Äquivalent an dreiwertigem aminartigem Stickstoff im Zwischenester einsetzt.

## Claims

1. A process for the production of cationic quaternary ammonium compounds containing at least one ester group corresponding to the following general formula:
R¹R²R³N⁺R⁴ A⁻
in which R¹, R² and R³ represent either
- an alkyl group of the formula CₙH₂ₙ₊₁, where n = 1 to 4, or
- a hydroxyalkyl group or a polyhydroxyalkyl group having the formula CₙH₍₂ₙ₊₁₋ₓ₎ (OH)ₓ, where n and x = 1 to 4, or
- an ester group having the formula R⁵COOR⁶-, where R⁵ is a C₆₋₂₂ alkyl or alkenyl group and R⁶ is a group corresponding to the following formula 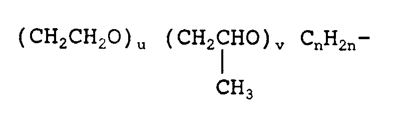 in which n = 1 to 4, u and v = 0 to 6 in any arrangement;
R⁴ is
- an alkyl group having the formula CₙH₂ₙ₊₁, where n = 1 to 4,
- a polyoxyalkylene chain corresponding to the formula 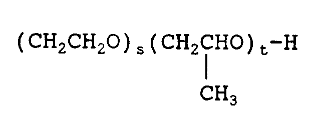 where s and t = 0 to 6; A⁻ is a counter anion which is either
- alkyl sulfate having the formula SO₄(CₙH₂ₙ₊₁)⁻, where n = 1 to 4;
- alkyl phosphate having the formula PO₄ (CₙH₂ₙ₊₁)₂⁻, where n = 1 to 4;
- the anion of an organic acid having the formula CₙH_{(2n-1-a-b)}(OH)ₐ (COOH)_{b}, where n = 1 to 4 and a, b = 1 to 3;
- a phosphate or phosphite anion; Cl⁻, Br⁻, CH₃C₆H₄SO₃⁻; the process being characterized in that it comprises the following steps:
a) esterification of a fatty acid having the formula R₅COOH with an alkanolamine having the formula R¹R²NR⁶-OH in the presence of a reducing agent from the group consisting of hypophosphorous acid, phosphorous acid, oxalic acid or alkali metal or alkaline-earth metal salts thereof or organic esters thereof or in the presence of a reducing agent from the group consisting of thioacetamine, hydrazine, hydroquinone and derivatives thereof and subsequent passage of a stream of air through the ester at a temperature of 40 to 50°C and in a ratio of air to ester of 10:1 to 0.5:1 and preferably in a ratio of 3:1 m³ air per t ester through the reaction mixture; and
b) alkylation of the resulting compound by a reagent having the formula R⁴ A or by an alkylene oxide from the group consisting of ethylene oxide, propylene oxide or mixtures thereof.

2. A process as claimed in claim 1, characterized in that the esterification is carried out at a temperature of 120 to 240°C, preferably at a temperature of 140 to 200°C and more preferably at a temperature of 160 to 180°C.

3. A process as claimed in claim 1, characterized in that the esterification is carried out under a pressure of 1.333 to 133.3 mbar, preferably under a pressure of 1.333 to 26.66 mbar and more preferably under a pressure of 1.333 to 6.665 mbar.

4. A process as claimed in claim 1, characterized in that the esterification is carried out under atmospheric pressure in the presence of an inert solvent.

5. A process as claimed in claim 4, characterized in that an aliphatic or aromatic hydrocarbon, such as for example toluene, xylene, n-heptane or n-decane, is used as the inert solvent.

6. A process as claimed in claim 1, characterized in that the esterification is carried out in the presence of an acidic catalyst, for example a sulfonic acid, such as p-toluene sulfonic acid or methane sulfonic acid, or phosphoric acid, phosphorous acid or hypophosphorous acid or oxalic acid or a Lewis acid, such as tin(II) salts for example.

7. A process as claimed in claim 6, characterized in that, based on the reaction mixture, 0.01 to 5% by weight, preferably 0.02 to 0.5% by weight and more preferably 0.05 to 0.15% by weight of reducing agent and catalyst is used.

8. A process as claimed in claim 1, characterized in that fatty acids of natural or synthetic origin or hydrogenation or hydrolysis products thereof are used.

9. A process as claimed in claim 1, characterized in that triethanolamine, methyl diethanolamine or adducts thereof with ethylene oxide and/or with propylene oxide is/are used as the amine.

10. A process as claimed in claim 1, characterized in that the reaction is conducted in such a way that the reaction mixture has a diester content of 60 to 80% by weight.

11. A process as claimed in claim 1, characterized in that a dialkyl sulfate or a trialkyl sulfate, preferably dimethyl sulfate, is used as the alkylating agent.

12. A process as claimed in claim 1, characterized in that fatty acid and amine are used in a ratio of 0.2 : 1 to 1 : 1, preferably in a ratio of 0.4 : 1 to 0.8 : 1 and more preferably in a ratio of 0.5 : 1 to 0.7 : 1 per equivalent alkanolamine.

13. A process as claimed in claim 11, characterized in that the alkylation is carried out at atmospheric pressure in the presence of a short-chain aliphatic alcohol, preferably isopropyl alcohol, in a quantity of 5 to 50% by weight, preferably in a quantity of 10 to 30% by weight and more preferably in a quantity of 15 to 25% by weight, based on the reaction mixture.

14. A process as claimed in claim 11, characterized in that the alkylating agent is used in a quantity of 0.5 to 1 mol, preferably in a quantity of 0.8 to 1 mol and more preferably in a quantity of 0.9 to 0.95 mol per equivalent trifunctional amine-like nitrogen in the intermediate ester.

## Revendications

1. Procédé de préparation de composés quaternaires cationiques de l'ammonium renfermant au moins un groupe ester de la formule générale:
R¹R²R³N⁺R⁴ A⁻;
dans laquelle R¹, R², R³ représentent soit:
- un groupe alkyle de la formule CₙH₂ₙ₊₁, dans laquelle n représente 1 à 4 dans chaque cas, ou
- un groupe hydroxyalkyle ou polyhydroxyalkyle de la formule CₙH₍₂ₙ₊₁₋ₓ₎(OH)ₓ, dans laquelle n et x représentent chacun 1 à 4, ou
- un groupe ester de la formule R⁵COOR⁶ -, dans laquelle R⁵ est un groupe alkyle ou alcényle comportant 6 à 22 atomes de carbone et R⁶ représente un groupe de la formule 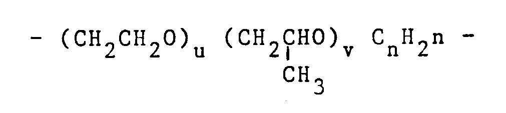 dans laquelle n représente 1 à 4 dans chaque cas, u et v correspondent chacun à 0 à 6, dans une disposition quelconque, et R⁴ est
- un groupe alkyle de la formule générale CₙH₂ₙ₊₁ dans laquelle n représente 1 à 4 dans chaque cas,
- une chaîne polyoxyalkylène de la formule 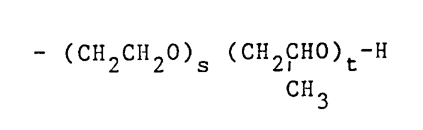 dans laquelle s et t représentent chacun 0 à 6; A- est un contre-anion , qui est au choix
- un alkylsulfate de la formule SO₄(CₙH₂ₙ₊₁)-, dans laquelle n représente 1 à 4 dans chaque cas;
- un alkylphosphate de la formule PO₄(CₙH₂ₙ₊₁)₂-, dans laquelle n représente 1 à 4 dans chaque cas;
- l'anion d'un acide organique de la formule CₙH_{(2n-1-a-b)} (OH)ₐ (COOH)_{b}, dans laquelle n représente 1 à 4 dans chaque cas, et a et b correspondent chacun à 1 à 3;
- un anion phosphate ou phosphite;
- Cl⁻; Br⁻, CH₃C₆H₄SO₃⁻;
ledit procédé étant caractérisé en ce qu'il comprend les étapes suivantes:
a) estérification d'un acide gras de la formule R₅COOH par une alcanolamine de la formule R¹R²NR⁶-OH, en présence d'un réducteur faisant partie du groupe constitué des acides hypophosphoreux, phosphoreux et oxalique ou de leurs sels de métaux alcalins ou alcalino-terreux ou de leurs esters organiques, ou d'un réducteur organique faisant partie du groupe de la thioacétamine, de l'hydrazine, de l'hydroquinone et de leurs dérivés, suivie de la conduite au travers de l'ester d'un courant d'air à une température de 40 à 50 °C et avec un rapport entre l'air et l'ester compris dans l'intervalle de 10:1 à 0,5:1, de préférence dans un rapport de 3:1 m3 d'air par tonne d'ester au travers du mélange réactionnel; et
b) alkylation du composé obtenu par un réactif de formule R⁴ A ou par un oxyde d'alkylène faisant partie du groupe constitué des oxydes d'éthylène et de propylène, ainsi que de leurs mélanges.

2. Procédé selon la revendication 1, caractérisé en ce que l'on opère l'estérification à une température comprise entre 120 et 240 °C, de préférence entre 140 et 200 °C et, en particulier, entre 160 et 180 °C.

3. Procédé selon la revendication 1, caractérisé en ce que l'on opère l'estérification à une pression comprise entre 1,333 et 133,3 mbars, de préférence entre 1,333 et 26,66 mbars et, en particulier, entre 1,333 et 6,665 mbars.

4. Procédé selon la revendication 1, caractérisé en ce que l'on opère l'estérification à la pression atmosphérique en présence d'un solvant inerte.

5. Procédé selon la revendication 4, caractérisé en ce que l'on utilise comme gaz inerte, un hydrocarbure aliphatique ou aromatique, tel que toluène, xylène, n-heptane ou n-décane.

6. Procédé selon la revendication 1, caractérisé en ce que l'on opère l'estérification en présence d'un catalyseur acide, par ex. un acide sulfonique tel l'acide p-toluènesulfonique, l'acide méthanesulfonique, l'acide phosphorique, l'acide phosphoreux, l'acide hypophosphoreux, l'acide oxalique ou un acide de Lewis, tel les sels bivalents d'étain.

7. Procédé selon la revendication 6, caractérisé en ce que l'on utilise une proportion de réducteur et de catalyseur par rapport au mélange réactionnel de 0,01 à 5 % en poids, de préférence de 0,02 à 0,5 % en poids et, en particulier, de 0,05 à 0,15 % en poids.

8. Procédé selon la revendication 1, caractérisé en ce que l'on utilise des acides gras d'origine naturelle ou synthétique ou leurs produits d'hydrogénation ou de décomposition.

9. Procédé selon la revendication 1, caractérisé en ce que l'on utilise comme amine, de la triéthanolamine, de la méthyldiéthanolamine ou leurs produits d'addition à l'oxyde d'éthylène et/ou de propylène.

10. Procédé selon la revendication 1, caractérisé en ce que l'on conduit la réaction de manière que le mélange réactionnel présente une concentration en diesters de 60 à 80 % en poids.

11. Procédé selon la revendication 1, caractérisé en ce que l'on utilise comme réactif alkylant, un dialkylsulfate ou un trialkylsufate, de préférence du diméthylsulfate.

12. Procédé selon la revendication 1, caractérisé en ce que l'on met en oeuvre l'acide gras et l'amine dans un rapport de 0,2:1 à 1:1, de préférence de 0,4:1 à 0,8:1 et, en particulier, de 0,5:1 à 0,7:1 par équivalent d'alcanolamine.

13. Procédé selon la revendication 11, caractérisé en ce que l'on opère l'alkylation à la pression atmosphérique en présence d'un alcool aliphatique à chaîne courte, de préférence de l'alcool isopropylique, qui est présent en proportion de 5 à 50 % en poids, de préférence de 10 à 30 % en poids et, en particulier, de 15 à 25 % en poids par rapport au mélange.

14. Procédé selon la revendication 11, caractérisé en ce que l'on met en oeuvre l'agent alkylant en proportion de 0,5 à 1, de préférence de 0,8 à 1 et, en particulier, dans une proportion de 0,9 à 0,95 mole par équivalent d'azote trivalent du type amine dans l'ester intermédiaire.
